# EUROPEAN PATENT APPLICATION

(11) **EP 3 590 501 A1**
(43) Date of publication of application: **08.01.2020**
(21) Application number: 18182170.3
(22) Date of filing: 06.07.2018
(51) Int. Cl.: A61K 9/14, B29C 64/106

(54) **MANUFACTURING PROCESS AND SYSTEM FOR MANUFACTURING A 3D PRINTED DRUG DELIVERY PRODUCT**

(71) Applicant: Nederlandse Organisatie voor toegepast- natuurwetenschappelijk onderzoek TNO, 2595 DA 's-Gravenhage (NL)
(72) Inventor: BROUWERS, Leonardus Antonius Maria, 2595 DA 's-Gravenhage (NL); HOPPENBROUWERS, Marcus Benedictus, 2595 DA 's-Gravenhage (NL); AULBERS, Antonius Paulus, 2595 DA 's-Gravenhage (NL); VERHOEVEN, Godefridus Hendrikus Willebrordus, 2595 DA 's-Gravenhage (NL)
(74) Representative: V.O.

(57) **Abstract**

In an aspect, an extrusion printer is provided comprising a print head comprising one or more nozzles suited for 3D printing of a pharmaceutical product. A metering pump is coupled to the print head and is arranged to control a flow to said print head and an extruder device comprises an input arranged to receive a powder or pellet material. A three-way pressure valve is fitted between the metering pump and the extruder device to automatically distribute a constant output flow from the extruder to an input flow directed towards the metering pump and a remainder flow directed towards the overflow outlet.

## Description

### FIELD OF INVENTION

The invention relates to the manufacture of a 3D printed product, in particular of a drug delivery product that is manufactured by a fused deposition modeling (FDM) process.

### BACKGROUND

In pharmaceutical formulation development, 3D printing is gaining increased attention as a strategy to overcome some challenges of conventional production of drug delivery products like oral dosage forms.

In conventional production many steps are involved like milling, mixing, granulation and compression. All those different steps can result in undesired variation of the quality of the final product with respect to drug loading, drug release, drug stability and pharmaceutical dosage form stability. The nature of these processes often requires that only large series of pills are produced efficiently at an acceptable cost. 3D printing has shown unprecedented flexibility in the design and manufacturing of complex objects which can be used in personalized and programmable medicine. By eliminating a number of sub processes 3D printing also brings the possibility of significant reduction in process control steps and related paperwork, thereby significantly reducing the manufacturing costs.

Pharmaceutical formulations generally consist of an excipient material which forms the body of the dosage form in which the Active Pharmaceutical Ingredient (API) is dispersed. One category of excipients suitable for FDM are thermoplastic polymer excipients. In pharma those excipients are generally used for processing by hot-melt extrusion (HME). Of particular interest is the use of HME to disperse active pharmaceutical ingredients (APIs) in a matrix at the molecular level, thus forming solid solutions. This method is becoming more and more important because the percentage of poorly soluble new chemical entities in drug development is constantly increasing. It is known that especially for BCS class II compounds, improved absorption and therapeutic efficacy can be realized by enhancing API solubility. The technology itself can be described as a process in which a mixture of excipient and API materials melts or softens under elevated temperature and pressure and is forced through an orifice by means of an extruder, which typically comprises a screw pump device.

In an other aspect the invention relates to deposition modeling of temperature sensitive food stuffs.

Appropriate thermoplastic behavior is a prerequisite of any polymer to be used in hot-melt extrusion. In extruded drug-delivery systems, larger quantities of polymer are required than when the polymer is used as a binder or coating agent. Consequently, it is crucial that the polymers be nontoxic and approved for human applications at high doses.

However, the number of such polymers approved for pharmaceutical use is limited and most of the candidates have limited mechanical properties not well suited for further processing.

Screw extruders are known from high volume plastic manufacturing industry. Although screw extruders are well capable of melting and mixing a range of starting ingredients they are accompanied with limitations. These include, among others, long equilibration times both in terms of output temperature as in terms of output composition. This makes it impractical to gain control over output parameters, such as flow rate, by changing extrusion process parameters, such as rotation speed and feed rate of the solid starting materials.

One particular 3D manufacturing process is a Fused Deposition Modeling or FDM process that can be used as a 3D printing technology. Typically, in a FDM a temperature-controlled print head extrudes a thermoplastic material layer by layer onto a build platform. Generally the material is fed to the print head in form of filament of wire, and for the hereinabove described Hot Melt Extrusion, it is thus natural that the filaments are fed into the FDM print head. Typically, metering may be provided by the feeding speed of the filament towards the print head. In order to improve control over the flow rate of a material to be printed, the metering pump may be used to start and stop a flow to the print head at will in an agile way that is needed for 3D printing at an acceptable speed. For example, enabling the termination of a first print sequence and initiating the start of a second, or varying the output in a more subtle manner to create more precise 3D printing structures. This variation should be fast enough to be compatible with the time scale of the printing process. However, there are several problems related to this method of feeding a filament to a FDM print head including:
i) the filaments used typically have poor mechanical properties which makes them difficult to handle in a FDM print head;
ii) the accuracy of the dosing of the FDM print head is very dependent on the accuracy of the diameter of the filament, which may not be easy to control with the available thermoplastic excipient materials;
iii) the accuracy of the API distribution in the pill is very dependent on the homogeneity of the dispersion of the API in the filament;
iv) execution of two separate melting steps increases the heat load on the materials, especially for pharmaceutical product this can lead to deterioration of the drug substance;
v) the separate melting steps may lead to segregation of components in a used mixture of ingredients which introduces uncertainty in the mixture composition of excipient and active product.
vi) The manufacturing of the filament from pellets or powder and the printing process are typically separate processes which may, especially in pharma application, result in a need for additional quality control steps.

DE102012000988 describes a FDM device for the formation of three dimensional plastic objects wherein objects are made in a continuous process comprising steps of filling a pressure volume with a melt, feeding the pressurized melt to an opening, and drop-wise release of the melt for constructing a three dimensional object. DE102012000988 does not provide for a way to control the residence time of the construction material within the heated system at times when the system is in non-steady-state operation, i.e. during start-up or at times when printing is paused or halted. In stead, closing the printing opening will lead to pressure build up in the system and an accompanied increased residence time of the construction material within the heated system. Upon re-starting of the system this will lead to poor droplet volume control, and more importantly, to uncontrolled mixing and temperature- or pressure induced degradation of the ingredients comprising the construction material.

In one aspect, the present invention aims, to address at least part of the above described problems by providing a 3D manufacturing method and a device for the manufacturing of pharmaceutical products with good control over the printing process, by accurate control over, and metering of, the process flow while maintaining a stable process of mixing and heating of the starting ingredients.

### SUMMARY OF THE INVENTION

In an aspect, a method is provided of manufacturing a pharmaceutical product, produced by a mixture of an excipient and an active pharmaceutical ingredient. The method comprises: receiving a mixture of powder or pellet material by an extruder device in a predefined ratio of excipient and active pharmaceutical ingredient for extruding as extruded product in a constant flow; distributing the constant output flow of the extruded product, from the extruder to an input flow directed towards a metering pump that is suited for regulated provision of the extruded product to a print head; printing a product by the print head comprising one or more nozzles suited for 3D printing of the product in a metered fashion from the flow received by the metering pump; and distributing part of the constant output flow from the extruder not provided to the print head to a remainder flow directed towards an overflow outlet. The constant output flow of the extruder is thereby arranged to provide a sufficient pressure to the input, e.g. higher than a threshold value of an inlet valve, of the metering pump thus ensuring that the metering pump is always filled. The remainder of the flow, which is the difference between the constant flow provided by the extruder and the varying flow taken by the metering pump, is directed towards the overflow outlet.

### BRIEF DESCRIPTION OF THE DRAWINGS

The invention will further be elucidated on the basis of exemplary embodiments which are represented in a drawing. The exemplary embodiments are given by way of non-limitative illustration. It is noted that schematic representations of embodiments of the invention that are given by way of non-limiting example.

In the drawings:
Fig. 1 illustrates a prior art FDM device;
Fig. 2 schematically depicts a method of manufacturing a pharmaceutical product according to the invention;
Fig. 3 illustrates a first exemplary embodiment of a device for manufacturing a pharmaceutical product according to the invention;
Fig. 4 illustrates a second exemplary embodiment of a device for manufacturing a pharmaceutical product according to the invention.

### DETAILED DESCRIPTION

In pharmaceutical formulation development, 3D printing is gaining increased attention as a strategy to overcome some challenges of conventional production of drug delivery products like oral dosage forms.

In conventional production methods many steps are involved like milling, mixing, granulation and compression. All of these different steps can introduce undesired variation in the quality of the final product with respect to drug loading, drug release, drug stability and pharmaceutical dosage form stability. The nature of these production methods often requires that only large series of pills are produced efficiently at an acceptable cost. 3D printing has shown unprecedented flexibility in the design and manufacturing of complex objects which can be used in personalized and programmable medicine. 3D printing also brings about the possibility of eliminating a number of sub process steps, resulting in significant reduction in process control steps.

It is an aspect of the present invention to provide a 3D printing system suitable for the production of 3D printed drug delivery products, such as oral dosage forms.

Fused deposition modeling (FDM) is a particular type of 3D modeling especially suited for the production of plastic 3D objects. Suitable conventional materials typically include thermoplastic polymers such as acrylonitrile butadiene styrene, thermoplastic polyurethane, polylactic acid, high-impact polystyrene, and polyamides (nylon). FDM devices are typically fed by filaments or wires formed of such raw materials. These filaments are introduced into the device by pulling them from a spool from which they are transported to a hot end of the device in which the raw material is liquefied and pushed towards an opening. This opening may be a hole at the end of a nozzle with a diameter that allows molten or liquefied material to exit the system. If nozzles are provided on a movable print head and/or material is deposited onto a computer movable building platform 3D objects may be formed. Typically 3D objects are made in a layer-wise fashion by depositing a continuous stream of material along a pre-programmed 2D trajectories. Typically the flow of material is interrupted and/or slowed down, e.g.: to allow removal of a finished product; to allow moving of the nozzle from an end point of a first trajectory to a starting point of a second trajectory; to allow moving of the nozzle from an end point after finishing printing of a first layer to a starting point for printing of a second layer; and/or to allow a slowing down of the print head during printing of, for example, corners in trajectories. The process can then be resumed to manufacture further identical or, if desired, different discrete products. To prevent solidification of material inside the device, all parts of the device that come in contact with the liquefied material flow, including nozzle and pressure chambers, if any, need to be heated. Geometry of the nozzle, pressure, and the melt behavior of the liquefied material flow are important parameters in the process.

In an embodiment of the present invention a fused deposition modeling method is provided for the manufacturing of 3D printed products.

In an embodiment of the present invention a fused deposition modeling method is provided for the production of 3D printed drug delivery products.

Pharmaceutical formulations generally consist of an excipient material which forms the body of the dosage form in which the Active Pharmaceutical Ingredient (API) is dispersed. One category of excipients suitable for FDM are thermoplastic polymer excipients. In pharma those excipients are generally used for processing by hot-melt extrusion (HME). Of particular interest is the use of HME to disperse active pharmaceutical ingredients (APIs) in a matrix at the molecular level, thus forming solid solutions. It is known that improved absorption and therapeutic efficacy can be realized by enhancing API solubility. The technology itself can be described as a process in which a mixture of excipient and API materials melts or softens under elevated temperature and pressure and is forced through an orifice by means of an extruder, which typically comprises a screw pump device.

In an embodiment of the present invention a fused deposition modeling method is provided for the production of 3D printed drug delivery products in which improved absorption and therapeutic efficacy can be realized. Good mixing of the API with the polymer excipient material is provided.

In an embodiment of the present invention a device is provided suited for fused deposition modeling for the production of 3D printed drug delivery products comprising thermosensitive active pharmaceutical ingredients.

In case FDM devices are used to produce 3D plastic components build from different raw materials in a single product devices can be used with multiple independent liquefied material streams and nozzles. By combining these streams, multi component objects may be formed. However, multiple streams of molten thermoplastic materials tend to mix poorly in the time-scale desired for printing.

Therefore, the use of pre-mixed raw materials may be preferred. In this case, for example, the FDM device may be fed with a pre-formed filament with the desired amounts and degree of mixing desired starting materials. This filament may be formed, for example, from a combination of, for example, any of the conventional polymers listed above to which additives such as colorants or fillers may be added.

Extrusion is a high-volume manufacturing process known for plastic materials, in which a continuous stream of liquefied building material is forced trough a narrow opening and fed into a cooling bath to form elongated filaments. A wide variety of materials may be used, including thermoplastic polymers or ceramics or even paste-like materials such as chocolate.

Extruders typically comprise a warm and a cold end. Cold raw material, in the form of pellets or powders, is fed from a hopper into an opening near the rear of an elongated barrel. Inside this barrel, raw material comes into contact with one or more screws that run along the length of the barrel. The rotating screw or screws force the raw material forward into the barrel where, heat generated by friction, in addition to externally applied heat, causes the raw materials to gradually melt and mix as they are processed towards an opening at the other end of the barrel. At this point, the molten flow is forced out an opening. Depending on the geometry and dimension of the opening, and by applying suitable cooling to the exiting process stream, filaments comprising a mixture of ingredients may be formed.

In extrusion processes, once steady state is reached, the output from an extruder is determined by the input flow of powder or pellets, and is independent from the speed of the extruder screw(s). Generally, extruder systems react very slowly on variations in process conditions and varying composition of the input. Upon disturbance a long time may be needed to reach steady state again.

In an embodiment of the present invention a method is provided for the production of 3D printed products in which a stable mixture formed from a number of raw starting materials, is provided to the printer over a long period of time, to facilitate the production of a series of products with minimal variation in composition and properties.

Mixing of raw starting materials may be performed in an extruder.

Mixing of the API and polymer excipient for the production of 3D printed drug delivery products may be performed in an extruder.

Although screw extruders are well capable of the melting and mixing of a range of starting ingredients they are accompanied with limitations. These include, among others, long equilibration times both in terms of output temperature as in terms of output composition. This makes it impractical to gain control over output parameters, such as flow rate, by changing extrusion process parameters, such as rotation speed and feed rate of the solid starting materials.

The number of suitable polymers approved for pharmaceutical use is limited and most of the candidates have limited mechanical properties not well suited for further processing.

One particular 3D manufacturing process is a Fused Deposition Modeling or FDM process that can be used as a 3D printing technology. In a temperature-controlled FDM print head a thermoplastic material is extruded onto a build platform to form products in a layer-by- layer way. Generally the material is fed to the print head in form of filament of wire, and for the hereabove described Hot Melt Extrusion, it is thus natural that the filaments are fed into the FDM print head. In order to improve control over the flow rate of a material to be printed, a metering pump may be added in between the exhaust of the extruder and the print head. Said metering pump may be used to start and stop a flow to the print head at will in an agile way that is needed for 3D printing at an acceptable speed. For example, enabling the termination of a first print sequence and initiating the start of a second, or varying the output in a more subtle manner to create more precise 3D printing structures. This variation should be fast enough to be compatible with the time scale of the printing process. There are several important limitations related to methods comprising feeding of a filament to a FDM print head.

A limitation of filament fed FDM methods involves the mechanical properties of the filament produced by the extruder. These need to be sufficient for handling in the print head. Poor mechanical properties, such as brittleness, make the filaments difficult to handle and results in poor performance of a FDM print head

A further limitation of filament fed FDM originates from the fact that the accuracy of the dosing of the FDM print head is very dependent on the accuracy of the diameter of the filament. For this reason filaments with uniform diameter along the length of the filament are needed. Poor mechanical properties in one of the filaments constituents may lead to formation of filament with to non-uniform diameter.

A further limitation of filament fed FDM methods relates to the accuracy of the ingredient distribution and variations therein. Good ingredient distribution across a series of manufactured products requires homogeneous dispersion of ingredients in the filament. This is especially important for drug dosage products.

An additional limitation relates to the fact that in filament fed FDM methods ingredients constituting the filament are exposed to two melting steps: one during filament formation, the other during filament processing. This results in the manufacture of products of which the ingredients are exposure to increased heat, or heat load. This may be especially problematic for temperature sensitive ingredients such as food stuffs or temperature sensitive active pharmaceutical ingredients. In drug delivery products, excessive heat load may lead to deterioration of the active pharmaceutical ingredient. In addition, the use of two separate melting steps may also cause segregation of substances which introduces uncertainty in the mixture composition of excipient and active product.

A further limitation of manufacturing of products, e.g. pharmaceutical products, by FDM printing is that FDM typically involves a multi step process, the manufacturing of the filament from pellets or powder and the printing process are typically separate processes. This may, especially in pharma application, result in a need for additional quality control steps.

It is a goal of the present invention to address the above mentioned aspects by providing a method of 3D manufacturing of products comprising a homogeneous mixture of ingredients without subjecting the raw materials to excessive heat load.

By directly introducing pellets and or powders of the desired ingredients the number of heating steps and heatload can be reduced. In one embodiment according to the present invention this is achieved by coupling the outlet an extruder to the inlet of a heated metering unit. In this way, well-mixed material is provided to the metering pump without the additional cooling and re-melting steps that would be needed in case material was fed as a filament.

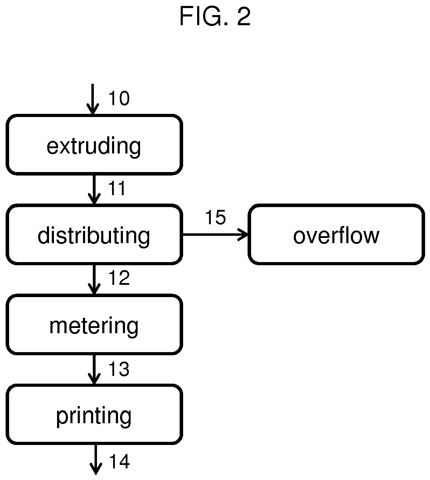

Fig. 2, schematically depicts an exemplary manufacturing method according to the invention. In the exemplary method a mixture of starting materials is extruded in an extruding step. This may involve an extruder unit provided with a single screw extruder. Raw material or mixtures of materials are fed to the barrel of said extruder. The barrel may be heated. Rotation of the screw mixes and transports the raw materials along the length of the tube towards the exit opening. As material is moved along the barrel, friction caused by rotation of the screw results in gradual mixing and melting of the process flow.

It is important to realize that the exit flow of an extruder is typically a constant volume flow. Also important to realize is that during operation of the extruder, the space between barrel wall and screw is generally not completely filled with a process flow; some open volume remains available. An interruption in the exit flow, for example caused by a temporary blockade, will lead to a pressure build-up in the barrel. At the end of the barrel a molten mixture of starting materials flows out of an exit opening. Once steady state operation is reached the composition of a molten flow of starting materials depends on the feeding ratio of raw starting materials. A long time may be required to reach a new steady state if, for example, the feeding ratio is changed.

After extruding, the method according to the invention comprises a distributing process. From an exit opening in the barrel the molten mixture of materials is transported to distributing member. This distributing member is suitable to distribute part of the incoming flow to a metering pump that is suited for regulated provision of the extruded product to a print head. The remainder of the extruded flow that is not provided to the print head, is directed towards an overflow outlet. By moving an unused portion of the extruded flow to an overflow outlet, pressure build up in the extruder is prevented and residence time of materials within the tube remains unaltered. This helps in preventing disturbances in the extrusion process and thus helps in maintaining a constant outflow of the extruder once a steady state is reached. The distributing member may also be used to direct extruded flow completely to the overflow outlet. This will, for example, be useful in events when the printing process is halted or in events when the extruder is not operating under steady state conditions. The distributing member may be a three-way pressure valve or any other type of valve suited for the above describe purpose.

The overflow outlet is connected to a waste outlet and may further be may be connected to a sampling port for sampling purposes such as quality control. Optionally, in case non-temperature sensitive ingredients are used, the overflow outlet may be fed back into the extruder. This reduces material losses. In case thermosensitive active pharmaceutical ingredients are used, loss of material is may be preferred over recycling which causes exposing the flow to an additional extruding cycle. In such event the overflow outlet is typically connected to a waste outlet.

The portion of the flow that is required for the printing process is fed to the inlet of a metering pump. This pump may be any type of pump suited to exert control over the flow rate of a material to be printed. The metering pump may be used to start and stop a flow to the print head at will in an agile way that is needed for 3D printing at an acceptable speed. For example, enabling the termination of a first print sequence and initiating the start of a second, or varying the output in a more subtle manner to create more precise 3D printing structures. This variation should be fast enough to be compatible with the time scale of the printing process.

From the metering pump the molten mixture of materials is directed to a printing unit. This may be a print head comprising one or more nozzles suited for 3D printing of the product in a metered fashion from the flow received by the metering pump.

Optionally, individual nozzles or a set of nozzles in a print head may be further provided with a metering unit. This allows for more precise control of an outflow out of a nozzle. Auger pumps are pumps in which material is displaced by a rotating screw. Archimedes-type screws form a suitable type of auger pumps. Alternatively, progressive cavity pumps that are characterized in that fluid transfers through the pump as the rotor is turned, through a sequence of small, fixed shape, discrete cavities, are suitable as well.

In order to prevent solidification of the molten flow of materials within the system all parts of the device that are in contact with materials to be printed may be kept at a suitable temperature..

### DETAILED DESCRIPTION OF FIGURES

It will be appreciated that the visualized stages of an exemplary production method of manufacturing a pharmaceutical product, or devices for manufacturing pharmaceutical products according to the invention, are not limited to the exemplary process or embodiments, nor to the used materials in these examples. Other production methods, for example, comprising further steps, or products using other materials, as well as devices further provided with components suitable for such steps or components are also envisioned.

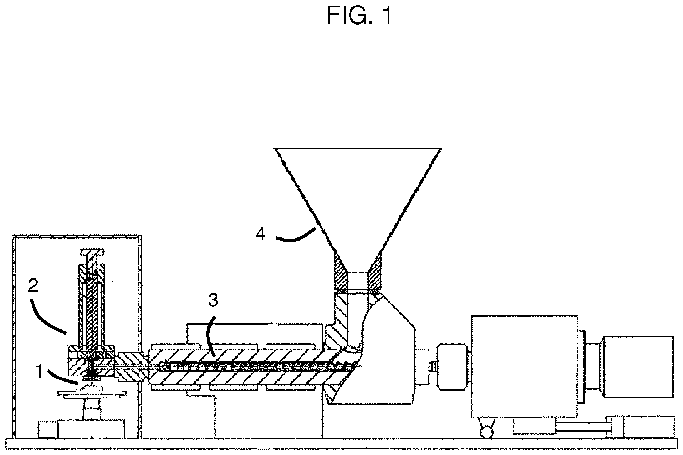

Fig.1 depicts a FDM device according to the teachings of DE102012000988. The device comprises: a print head 2 provided with a nozzle 1 from which material is printed; an extruder unit 3 with a barrel provided with a single screw; and a hopper 4 from which material to be printed is fed to the extruder. In the device described in DE102012000988 a molten flow of material is fed from the extruder directly to a print head from which material is deposited. Absent a distributing unit, the complete output stream of the extruder is directed to the print head. Absent a metering unit the flow of this stream is not regulated and variations in flow speed and or composition in an exit flow from the extruder are directed towards the print unit. Reversely, variations in deposition rate caused by the print head, are transposed onto the extruder. For example, an interrupted output flow from the printhead will lead to a pressure build-up in in the extruder, causing disturbances in the processing conditions which leads to variations in composition of the material to be printed.

Fig. 2 depicts an exemplary schematic overview of a production method according to an aspect of the present invention. In the method a mixture of materials to be printed is fed 10 to an extruding unit. For the manufacturing of a pharmaceutical product the mixture comprises a mixture of an excipient and an active pharmaceutical ingredient. Other ingredients, such as food stuffs may be used as well. In the extruding unit the mixture of starting materials is mixed and melted. A molten flow 11 exiting the extruder is fed to a distributing unit. This unit is suitably selected to distribute the output flow of the extruded product, from the extruder to an input flow 12 that is directed towards a metering pump that is suited for regulated provision of the extruded product to a print head. The part of the constant output flow from the extruder that is not provided to the print head is distributed to a remainder flow 15 directed towards an overflow outlet.

The metering pump that is suited for regulated provision of the extruded product to a print head provides a flow 13 to said print head from which a flow 14 is ejected to form the desired product. By providing a method comprising a distributing unit connected to an overflow outlet, part of the flow that is not used in the printing process can be directed to a waste outlet. This prevents pressure build up in the extruder unit and facilitates maintaining a steady state output of said extruder. Directing part the unused portion of the extruder output flow to a waste outlet also facilitates maintaining a constant dwell time of components in the extruder. In this way a constant heat load is provided to the materials during the extrusion process. Both of the afore mentioned effects help in maintaining a constant composition and quality of the manufactured products.

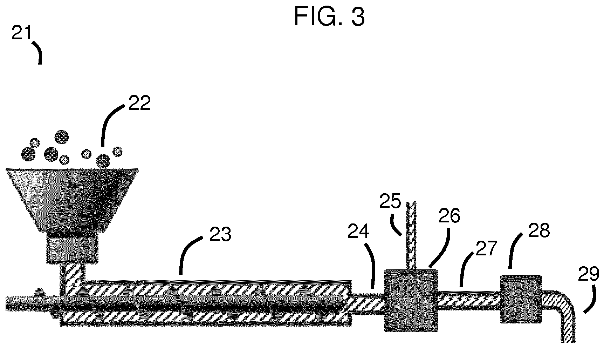

Fig. 3 schematically depicts a first exemplary embodiment of a device 21 for manufacturing a pharmaceutical product, produced by a mixture 22 of an excipient and an active pharmaceutical ingredient. The exemplary device comprises an extruder device 23 comprising a barrel provided with a single screw. The exemplary extruder is suited to receive a mixture of powder or pellet material in a predefined ratio of excipient and active pharmaceutical ingredient for extruding as extruded product in a constant flow. From an exit opening the extruded product 24 is fed to an inlet of a distributing member 26 provided between the extruder device and the metering pump 28. The distributing device is suited for distributing the part of the constant output flow of the extruded product from the extruder that is needed in the printing process 27 to an input of the metering pump 28. The remainder flow 25, i.e. the part of the constant output flow from the extruder that is not provided to the print head, is directed towards an overflow outlet. In the first exemplary embodiment, shown in Fig. 3, the distributing member comprises a 3-way pressure valve.

The flow needed for the printing process is fed to the inlet of a metering pump that is suited for regulated provision of the extruded product to a print head 29. In the exemplary embodiment depicts a print head comprising one nozzle, suited for 3D-printing of the product in a metered fashion from the flow received by the metering pump. It will be appreciated that print heads comprising multiple nozzles, or alternatively multiple print heads, each comprising one or more nozzles are also envisioned. It will likewise be appreciated that products forms from other ingredients, such as food stuffs, are envisioned as well.

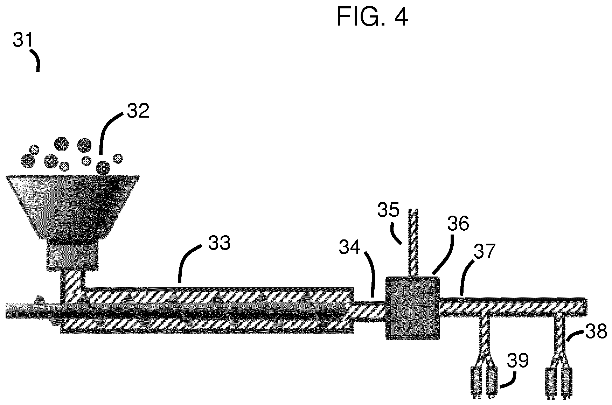

Fig. 4 schematically depicts a second exemplary embodiment of a device 31 for manufacturing a pharmaceutical product, produced by a mixture 32 of an excipient and an active pharmaceutical ingredient. The exemplary device comprises an extruder device 33 comprising a barrel provided with a single screw. The exemplary extruder is suited to receive a mixture of powder or pellet material in a predefined ratio of excipient and active pharmaceutical ingredient for extruding as extruded product in a constant flow. From an exit opening the extruded product 34 is fed to an inlet of a distributing member 36 provided between the extruder device and the inlet of a print head 38. The distributing device is suited for distributing the part 37 of the constant output flow of the extruded product from the extruder that is needed in the printing process to the inlet of said print head. The remainder flow 35, i.e. that part of the constant output flow from the extruder that is not provided to the print head, is directed towards an overflow outlet.

In the second exemplary embodiment the distributing member comprises a 3-way pressure valve. Optionally pressure regulating members such as a member comprising a volume and movable piston may be provided between the distribution member and a print head. In the exemplary second embodiment the device is provided with two print heads 38, each head comprising two nozzles fed by metering pumps 39, suited for 3D printing of the product in a metered fashion from the flow received from the pressure regulating member. In the second exemplary embodiment according to the invention each nozzle is provided with an individual metering pump. This has the added benefit that material flow can be regulated and tailored, for example to specific needs of each nozzle. Any suitable pump for metering such a flow can be used. Suitable pumps may for example include gear pumps or Archimedes type screw pumps such as Auger pumps or a progressive cavity pumps.

It will be appreciated that during the printing process the output flow from the extruder is preferably larger than the flow required, e.g. directed, by the metering pump during this process. The remainder flow, if any, may be directed to the waste outlet. Having the output flow of the extruder larger than the flow directed towards the metering pump flow may contribute to avoiding disturbances in the printing process, e.g. temporary halting of deposition of material.

It will be appreciated that embodiments with a different number of print heads and or print heads comprising a different number of nozzles, or print heads with multiple nozzles fed from a single metering pump as well as embodiments provided with other pressure regulating members are also envisioned.

It will likewise be appreciated that products formed from other ingredients, such as food stuffs, are envisioned as well.

## Claims

1. A method of manufacturing a pharmaceutical product, produced by a mixture of an excipient and an active pharmaceutical ingredient, comprising:
- receiving a mixture of powder or pellet material by an extruder device in a predefined ratio comprising excipient and active pharmaceutical ingredient for extruding as extruded product in a constant flow;
- distributing the constant output flow of the extruded product, from the extruder to an input flow directed towards a metering pump that is suited for regulated provision of the extruded product to a print head;
- printing a product by the print head comprising one or more nozzles suited for 3D printing of the product in a metered fashion from the flow received by the metering pump; and
- distributing part of the constant output flow from the extruder not provided to the print head to a remainder flow directed towards an overflow outlet.

2. A method according to claim 1, wherein the flow of the extruded product is interrupted depending on quality control parameters of the remainder flow.

3. A method according to claim 1, wherein said remainder flow is at least in part distributed to a waste.

4. A method according to claim 1 in which said APIs comprise a thermosensitive API.

5. A device for manufacturing a pharmaceutical product, produced by a mixture of an excipient and an active pharmaceutical ingredient, comprising:
- an extruder device suited to receive a mixture of powder or pellet material in a predefined ratio of excipient and active pharmaceutical ingredient for extruding as extruded product in a constant flow;
- a metering pump that is suited for regulated provision of the extruded product to a print head;
- a print head comprising one or more nozzles suited for 3D printing of the product in a metered fashion from the flow received by the metering pump; and
- a distributing member provided between the extruder device and the metering pump, suited for distributing the constant output flow from the extruder not provided to the print head to a remainder flow directed towards an overflow outlet; and to distributing the constant output flow of the extruded product from the extruder to an input of the metering pump.

6. A device according to claim 5 wherein the distribution member comprises a three-way pressure valve fitted between the metering pump and the extruder device to automatically distribute the constant output flow from the extruder to an input flow directed towards the metering pump and to a remainder flow directed towards the overflow outlet.

7. A device according to claim 6, wherein said remainder flow through the overpressure outlet is at least in part distributed to a waste outlet.

8. A device according to claim 6, wherein said remainder flow through the overpressure outlet is at least in part distributed to a sampling port suited for sampling for quality control purposes.

9. A device according to claim 5, in which said metering pump is a gear pump.

10. A device according to claim 5-8, wherein said metering pump is an auger pump.

11. A device according to claim 10, wherein said auger pump is an Archimedes type screw pump or a progressive cavity pump.
